Europäisches Patentamt

European Patent Office

Office européen des brevets

(19)

(11) Publication number: **0 249 498**
A1

(12) **EUROPEAN PATENT APPLICATION**

(21) Application number: **87305250.0**

(22) Date of filing: **12.06.87**

(51) Int. Cl.⁴: **C 07 D 251/32**
C 07 B 37/10, F 28 D 15/00

(30) Priority: **12.06.86 JP 137645/86**

(43) Date of publication of application:
**16.12.87 Bulletin 87/51**

(84) Designated Contracting States:
**DE ES FR GB IT NL**

(71) Applicant: **SHIKOKU CHEMICALS CORPORATION**
**147-1-Minato-machi Marugame-shi**
**Kagawa-ken (JP)**

(72) Inventor: **Hashimoto, Masayoshi 17-6, aza Teraura**
**Nakamura Kitajima-cho**
**Itano-gun Tokushima-ken (JP)**

**Takata, Mamoru**
**7-7, Takabou Tsutsushita Kitajima-cho**
**Itano-gun Tokushima-ken (JP)**

**Kabuki, Kiyoshige 43-14,aza Kamiyashiki**
**Kitamura Kitajima-cho**
**Itano-gun Tokushima-ken (JP)**

**Hamazaki, Makoto 40-1, aza Kowake**
**Ejiri Kitajima-cho**
**Itano-gun Tokushima-ken (JP)**

(74) Representative: **Senior, Alan Murray et al**
**J.A. KEMP & CO 14 South Square Gray's Inn**
**London WC1R 5EU (GB)**

(54) Apparatus for preparing cyanuric acid.

(57) Disclosed is an apparatus for preparing cyanuric acid by heating a molten metal (2) placed in a reaction vessel (1), supplying urea to the surface of the molten metal (2) and withdrawing a reaction product formed by thermal decomposition in the form of a plate, wherein a plurality of U-figured heating pipes (6), each having ends opened outside of the side wall (11, 12) of the reaction vessel (1), are buried in the molten metal (2) in the reaction vessel (1), and a burner (7) is connected to one open end of each heating pipe (6).

If this apparatus is used, the productivity is prominently increased and the continuous operation can be performed stably on an industrial scale.

*Fig. 1*

**Description**

APPARATUS FOR PREPARING CYANURIC ACID

Background of the Invention

(1) Field of the Invention

The present invention relates to an apparatus for preparing cyanuric acid by thermally decomposing urea on an industrial scale. Cyanuric acid is widely used as an intermediate for manufacturing an organic chlorine type bleaching fungicide or a flame retardant for a synthetic resin.

(2) Description of the Prior Art

In the process for preparing cyanuric acid by thermally decomposing urea, after urea is molten at a temperature of about 132°C, the viscosity of the melt is gradually increased and the melt becomes highly viscous like a thick melt syrup, and finally, the melt adheres in the form of a lump to the hot surface of a reaction vessel. This lump greatly inhibits conduction of heat and the heating temperature in the reaction system becomes uneven, and once formed cyanuric acid is further decomposed to cyanic acid gas, resulting in reduction of the yield of cyanuric acid. Furthermore, a large labor is necessary for removing the reaction product stuck to the heat transfer surface. Accordingly, it is very difficult to carry out the above-mentioned process continuously in a good yield on an industrial scale at a high efficiency.

As means for moderating this difficulty, we previously proposed a process and apparatus in which urea is thermally decomposed on a molten metal and a reaction product composed mainly of cyanuric acid is recovered in the form of a plate (see U.S. Patent No. 3,275,631. and U.S. Patent No. 3,524,853).

In the case where a molten metal is used as the heating medium as described above, since the difference of the specific gravity between the molten metal and the reaction product is large and the molten metal has a very large surface tension and a good heat conductivity, the reaction product can be solidified in the form of a continuous plate on the surface of the molten metal and the operation can be smoothly performed.

As means for imparting reaction heat to the molten metal as the heating medium in the above-mentioned preparation apparatus, there are arranged many electric heaters protected with quartz tubes in the reaction vessel.

In the apparatus comprising many electric heaters arranged as the heat source, the temperature of the molten metal can be controlled very easily, and therefore, a series of operations of melting starting urea supplied to the surface of the molten metal, increasing the viscosity of the melt and solidifying the melt in the form of a plate can be performed relatively smoothly. However, in the apparatus comprising electric heater as the heat source, the quantity of heat generated from the heat source is limited by the material and structure of the heater, and in the case where the electric heater is arranged in the reaction vessel, since the insulated portion of the electric heater is located in the vicinity of the side wall, as the starting urea-supplying portion, of the reaction vessel and sufficient heat cannot be supplied to the portion where a largest quantity of heat is necessary, the yield of cyanuric acid per unit surface area of the molten metal is relatively small, and the manufacturing cost is increased because of a large power consumption.

Summary of the Invention

Under this background, we made research with a view to increasing the productivity in the reaction apparatus for preparing cyanuric acid by thermally decomposing urea on the surface of a molten metal. As the result, we have now completed the present invention.

In accordance with one fundamental aspect of the present invention, there is provided an apparatus for preparing cyanuric acid by heating a molten metal placed in a reaction vessel, supplying urea to the surface of the molten metal and withdrawing a reaction product formed by thermal decomposition in the form of a plate, wherein a plurality of U-figured heating pipes, each having ends opened outside of the side wall of the reaction vessel, are buried in the molten metal in the reaction vessel, and a bunner is connected to one open end of each heating pipe.

In accordance with another aspect of the present invention, there is provided an apparatus for the preparation of cyanuric acid, which comprises a reaction vessel having a pair of side walls, a front wall and a rear wall, a molten metal layer contained in the reaction vessel and having a free liquid surface on the top thereof, which is disposed to convert urea to cyanuric acid by thermal decomposition on said liquid surface, a heating mechanism arranged in the side walls of the reaction vessel to extend through the interior of the molten metal layer and heat the molten metal by conduction of heat, a starting material supply mechanism arranged to supply urea to the liquid surface of the metal layer on the side of the front wall of the reaction vessel, and a product withdrawal mechanism arranged on the side of the rear wall of the reaction vessel to withdraw a plate-shaped cyanuric acid product, wherein the heating mechanism comprises a plurality of U-figured pipes, each having an inlet and an outlet, which are fixed to one side wall of the reaction vessel, straight portions extending vertically to the side walls and a curved portion located on the inner side of the other side wall with a small clearance therefrom, and burners arranged in inlet side openings of the respective pipes.

Brief Description of the Drawings

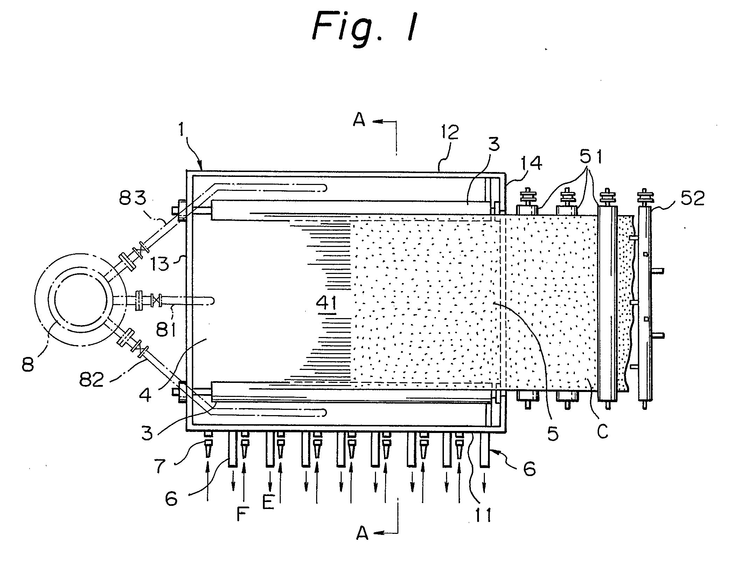

Fig. 1 is a plane view illustrating an embodiment of the apparatus of the present invention.

Fig. 2 is a view showing the section taken along the line A-A in Fig. 1.

Fig. 3 is a view showing the section taken along the line B-B in Fig. 2.

In the drawings, reference numeral 1 represents a reaction vessel, reference numeral 2 represents a molten metal, reference numeral 3 represents a rotary roll, reference numeral 6 represents a heating pipe, and reference numeral 7 represents a burner.

Detailed Description of the Preferred Embodiment

In carrying out the present invention, a gas burner or an oil burner can be used as the burner connected to the opening of the heating pipe. However, it is preferred that a gas burner in which combustion can be easily adjusted be used and a mixture of a fuel gas and air jetted from the burner be burnt in the heating pipe. The heating pipe can be arranged in multiple stages in the reaction vessel.

If necessary, an electric heater in which the temperature can be easily adjusted can be arranged in combination with this heating mechanism to control the temperature of the molten metal.

In the case where one heating pipe is arranged in the reaction vessel to meander over the entire region of the molten metal bath and the molten bath is heated by a combustion gas of a burner connected to one open end of this heating pipe, cyanuric acid can be formed by thermal decomposition of starting urea, but the difference of the temperature of the combustion gas between the inlet and outlet of the heating pipe is large and thermal decomposition of starting urea is locally accelerated or delayed. Accordingly, when the viscous reaction intermediate is gradually solidified, breakage of the plate-like reaction product is readily caused and continuous withdrawal of the product is often inhibited.

As typical instances of the molten metal to be used in the present invention, there can be mentioned elementary metals such as tin, bismuth and lead, and alloys such as an alloy consisting of tin, antimony and lead and an alloy consisting of bismuth, cadmium, lead and tin. These metals should always be maintained in the molten state at 230 to 350°C. If temperature of the molten metal exceeds 350°C, formed cyanuric acid is sublimated or decomposed to cyanuric acid.

In the apparatus of the present invention, there may be adopted a method in which a rotary roll is supported along the inner side wall surface of the reaction vessel and the bottom portion of the rotary roll is immersed in the molten metal, and the rotary roll is internally rotated at a peripheral speed of 1 to 5 m/min, whereby the starting material can be supplied between the inner wall of the reaction vessel and the rotary roll. Starting urea may be supplied in the solid state. Alternatively, urea may be heated at 150 to 180°C in advance and urea in the liquid state may be supplied to the surface of the molten metal, or starting urea may be subjected to ammonia-removing reaction and biuret-containing urea may be supplied to the surface of the molten metal, whereby the reaction speed can be further increased.

The content of unreacted urea in the plate-like reaction product withdrawn from the apparatus of the present invention is much lower than in the product obtained by the conventional apparatus.

However, it is preferred that the plate-like reaction product be pulverized to the powdery or granular state and then be charged into a stirring type heater and heated again at a temperature of 230 to 300°C to complete the reaction.

Ammonia gas and sublimated urea formed at the thermal decomposition of starting urea should be recovered, respectively. Urea sublimated during the reaction is absorbed and recovered in an aqueous solution of urea, whereby recovered urea can be used for the reaction again.

The combustion waste gas discharged from each heating pipe of the apparatus of the present invention is preferably recovered and used as the heat source for the step of purifying cyanuric acid by heating it in an aqueous solution of a mineral acid such as sulfuric acid or hydrochloric acid.

In the apparatus of the present invention, heating pipes are arranged at such positions that they are buried in the molten metal in the reaction vessel, and burners are connected to open ends of the heating pipes. Accordingly, a large quantity of heat from a hot gas generated by combustion of a fuel gas or oil can be conducted to the molten metal. In this combustion gas, there is produced such a large temperature gradient that the temperature of the combustion gas on the inlet side is 1300 to 1400°C and the temperature of the combustion gas on the outlet side is 500 to 600°C. In the present invention, however, since a plurality of U-figured heating pipes are arranged so that the pipe ends are opened outside of the side wall of the reaction vessel, high-temperature going portions and low-temperature returning portions of the heating pipes are brought close to each other and the heat quantity is equalized, and the quantity of heat conducted to the molten metal is substantially equal at all of the points orthogonal to the stream of the reaction product and the degree of advance of the reaction of supplied urea is substantially equal at all of the points orthogonal to the stream of the reaction product. Accordingly, the plate-like form is uniform in the product and the reaction is sufficiently advanced to reduce the content of unreacted urea in the plate-like reaction product and increase the hardness of the reaction product. Therefore, occurrence of troubles owing to breaking of the plate can be completely avoided. Furthermore, since the heating pipes having open ends fixed to the side wall of the reaction vessel are U-figured pipes, even if the heating pipes are heated, no heat stress is produced at attaching portions of the heating pipes. Therefore, the operation and stopping can be repeated very easily.

Example

The reaction apparatus shown in the drawings is used. Tin as the molten metal 2 is charged in a depth of 25 cm in an iron reaction vessel 1 of a rectangular parallelepiped shape having a floor area of 1 m² and a depth of 30 cm. Rotary rolls 3 are arranged at positions close to both the side walls of the reaction vessel so that the lower portions of the rotary rolls 3 are immersed in the molten metal 2. The rotary rolls 3 are internally rotated at a peripheral speed of 3 m/min by an electric motor through a reduction gear mechanism. In this cyanuric acid-preparing apparatus, between a starting material supply portion 4 on one end of the reaction vessel 1 and a reaction product withdrawal portion 5 on the other end of the reaction vessel 1, there are disposed eight U-figured heating pipes 6 having ends opened outside of the side wall of the reaction vessel 1. A diffusion combustion type gas burner 7 is connected to one open end of each heating pipe 6. A gaseous mixture of LPG gas and air blown from the burners 7 is burnt in the heating pipes 6.

The structure of this apparatus will now be described in detail. The reaction vessel 1 has confronting side walls 11 and 12, a front wall 13, a rear wall 14 and a bottom 15. The top portion of the vessel 1 is covered with a lid (not shown), and an exhaust opening (vent) is formed in the lid to discharge gases generated by thermal decomposition or urea, such as ammonia.

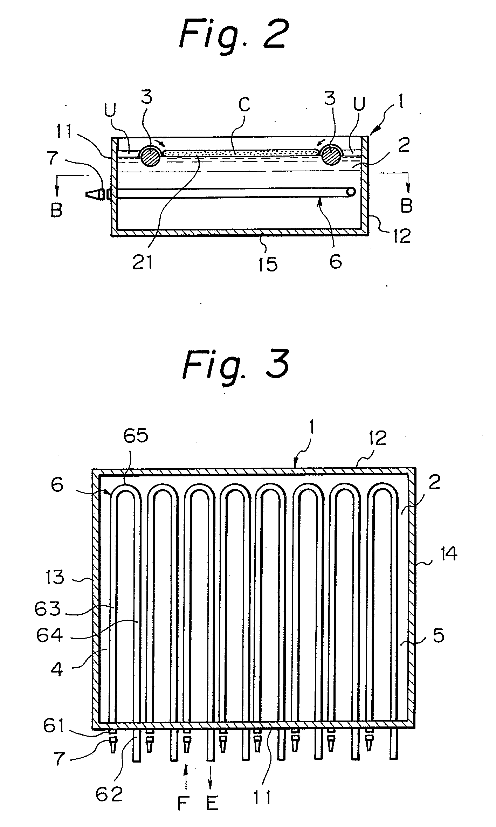

The molten metal 2 is contained within the reaction vessel 1 and a free liquid surface 21 is formed on the top of the molten metal 2. The starting urea supply portion 4 is present on the top surface of the molten metal layer 2 on the side of the front wall and the product withdrawal portion 4 is present on the top surface of the molten metal layer 2 on the side of the rear wall of the reaction vessel 1. A zone 41 for thermal decomposition of urea is formed between the starting urea supply portion 4 and the product withdrawal portion 5. A pair of rotary rolls 3 are arranged with small clearances from the side walls 11 and 12, respectively, of the reaction vessel 1 in parallel to these side walls 11 and 12, so that the lower portions of the rolls 3 are immersed in the molten metal layer 2 but the upper portions of the rolls 3 are located above the liquid surface 21. As shown in Fig. 2, the rotary rolls 3 are rotated internally, seen from above.

A urea tank 8 is arranged in the supply portion 4, and urea heated and molten is stored in this tank 8. This tank 8 comprises a urea conduit 81 for supplying molten urea U (see Fig. 2) onto the liquid surface of the molten metal between the rotary rolls 3, a conduit 82 for supplying molten urea U onto the liquid surface between the rotary roll 3 and the side wall 11 and a conduit 83 for supplying molten urea U onto the liquid surface between the rotary roll 3 and the side wall 12. Molten urea U supplied onto the liquid surface between the rotary rolls 3 is uniformly expanded and is gradually moved toward the withdrawal portion 5. On the other hand, molten urea U supplied onto the liquid surface between the side wall 11 (12) and the roll 3 is caused to flow into the liquid surface between the rotary rolls 3 with the internal rotation of the roll 3.

In the zone 41 for thermal decomposition of urea, the following reaction is caused
$$3(NH_2)_2CO \rightarrow C_3N_3O_3H_3 + 3NH_3$$
As means for withdrawing a formed plate C (see Fig. 2) of cyanuric acid at the withdrawal portion 5, there are disposed a series of delivery rollers 51 outside of the rear wall 14 of the reaction vessel 1, and the plate-like product C is withdrawn from the surface of the molten metal and the cooled plate-like product C of cyanuric acid is granulated or pulverized by a crusher 52.

Each of a plurality of the heating pipes 6 used in the apparatus of the present invention consists of a U-figured pipe having an inlet 61 and an outlet 62, which are fixed to one side wall 11, straight going and returning portions 63 and 64 vertical to the side wall 11, and a curved portion 65 located on the inner side of the other side wall 12 with a small clearance therefrom. As clearly shown in Fig. 3, a plurality of the U-figured pipes 6 are arranged in one plane parallel to the liquid surface 21 of the molten metal so that the going portions 63 and the returning portions 64 are alternatively located.

The burner 7 is arranged in the opening of the inlet 61 of each U-figured pipe 6. A combustion gas generated from a fuel F supplied to the burner 7 heats the molten metal 2 by conduction of heat while it passes through the going portion 63 and returning portion 64, and the exhaust gas E is discharged from the outlet 62.

The molten metal in the reaction vessel is heated at a temperature of 270 to 280°C and molten urea heated at 160°C is supplied at a constant rate of 150 kg/hour from the urea tank 8 to the front end portion of the reaction zone formed between the rotary rolls 3. Molten urea supplied into the reaction zone is spread on the surface of the molten metal in a thickness of 15 mm, and molten urea becomes gradually viscous while generating ammonia gas and within 3 minutes from the point of supply, molten urea is solidified in the form of a plate. The plate-like reaction product is continuously withdrawn from the withdrawal portion 5 of the reaction vessel.

Even if the above operation is continued for 24 hours, occurrence of a trouble such as adhesion of the reaction product to the wall surface of the reaction vessel or breaking of the plate-like reaction product is not caused at all.

Since the plate-like reaction product withdrawn from the reaction vessel contains about 8% of unreacted urea, the reaction product is crushed by a pulverizer, charged in a jacket type heater equipped with a stirrer, heated at a temperature of 290°C for 60 minutes and boiled in a 20% aqueous solution of sulfuric acid for 6 hours to purify the reaction product.

During the operation, starting urea is supplied in an amount of 3600 kg as a whole and the amount of formed cyanuric acid is 2215 kg and this amount corresponds to 85.9% of the theoretical yield.

Referential Example

The treatment is carried out in the same manner as described in the example except that seven 7-kw electric heaters covered with a quartz pipe are equidistantly arranged between the starting material supply portion and the reaction product withdrawal portion instead of the heating pipes having a gas burner attached thereto. When the urea supply rate is 150 kg/hour as in the example, the temperature of the molten metal is reduced to 260 to 270°C, and the reaction product-solidifying zone is shifted to the withdrawal side and the content of unreacted urea in the plate-like reaction product is elevated to 12%.

Moreover, the hardness of the reaction product is reduced, and when the reaction product is withdrawn, breaking of the plate is caused 5 times during 24 hours. Accordingly, the continuous operation is impossible.

When the supply rate of urea is reduced to 100 kg/hour, breaking of the plate-like reaction product is not caused. However, the total amount of urea supplied during the continuous operation conducted for 24 hours is 2400 kg and the amount of refined and recovered cyanuric acid is only 1450 kg (the yield is 84.3%)

According to the preparation apparatus of the present invention, when cyanuric acid is prepared by thermally decomposing urea on the surface of a molten metal, the amount of cyanuric acid produced per unit surface area of the molten metal can be increased by about 50% as compared with the amount of cyanuric acid prepared by the apparatus in which the molten metal is heated by an electric heater. Moreover, occurrence of breaking of the plate-like reaction product can be completely prevented and the continuous operation can be performed stably. Furthermore, since a cheap fuel gas or oil can be used as the fuel, an especially prominent effect can be attained when the present invention is carried out on an industrial scale.

## Claims

1. An apparatus for preparing cyanuric acid by heating a molten metal placed in a reaction vessel, supplying urea to the surface of the molten metal and withdrawing a reaction product formed by thermal decomposition in the form of a plate, wherein a plurality of U-figured heating pipes, each having ends opened outside of the side wall of the reaction vessel, are buried in the molten metal in the reaction vessel, and a burner is connected to one open end of each heating pipe.

2. An apparatus for preparing cyanuric acid according to claim 1, wherein the reaction vessel has a rectangular parallelepiped shape and a pair of rotary rolls are arranged along both the side walls of the reaction vessel so that the lower portions of the rotary rolls are immersed in the molten metal.

3. An apparatus for preparing cyanuric acid according to claim 1 or 2 wherein a urea tank is disposed to store urea heated in the molten state, and conduits are disposed to supply molten urea between a pair of rotary rolls, between one rotary roll and one side wall of the reaction vessel and between the other rotary roll and the other side wall of the reaction vessel, respectively.

4. An apparatus for preparing cyanuric acid according to claim 1, 2 or 3 wherein a gas burner is used as the burner.

5. An apparatus for preparing cyanuric acid according to claim 1, 2, 3 or 4, wherein tin is used as the molten metal and molten tin is maintained at a temperature of 230 to 350°C.

6. An apparatus for the preparation of cyanuric acid, which comprises a reaction vessel having a pair of side walls, a front wall and a rear wall, a molten metal layer contained in the reaction vessel and having a free liquid surface on the top thereof, which is disposed to convert urea to cyanuric acid by thermal decomposition on said liquid surface, a heating mechanism arranged in the side walls of the reaction vessel to extend through the interior of the molten metal layer and heat the molten metal by conduction of heat, a starting material supply mechanism arranged to supply urea to the liquid surface of the metal layer on the side of the front wall of the reaction vessel, and a product withdrawal mechanism arranged on the side of the rear wall of the reaction vessel to withdraw a plate-shaped cyanuric acid product, wherein the heating mechanism comprises a plurality of U-figured pipes, each having an inlet and an outlet, which are fixed to one side wall of the reaction vessel, straight portions extending vertically to the side walls and a curved portion located on the inner side of the other side wall with a small clearance therefrom, and burners arranged in inlet side openings of the respective pipes.

7. An apparatus for preparing cyanuric acid according to claim 6, wherein a plurality of the U-figured pipes are arranged in one plane parallel to the free liquid surface of the molten metal so that going and returning straight portions of the U-figured pipes are alternately located.

Fig. 1

0249498

# Fig. 2

# Fig. 3

| DOCUMENTS CONSIDERED TO BE RELEVANT | | | EP 87305250.0 | |
|---|---|---|---|---|
| **Category** | Citation of document with indication, where appropriate, of relevant passages | **Relevant to claim** | **CLASSIFICATION OF THE APPLICATION (Int. Cl 4)** | |
| Y | DE - B - 1 795 202 (SHIKOKU KASEI)<br>* Claim 1 *<br>-- | 1 | C 07 D 251/32<br>C 07 B 37/10<br>F 28 D 15/00 | |
| Y,D | DE - B - 1 260 475 (SHIKOKU KASEI)<br>* Claim 1 *<br>-- | 1 | | |
| Y | DE - B - 1 065 420 (BASF)<br>* Claim *<br>-- | 1 | | |
| Y | DE - B - 1 085 533 (BASF)<br>* Claim *<br>-- | 1 | | |
| A | ULLMANNS ENCYKLOPÄDIE DER TECH-nischen Chemie, 4th edition, vol. 2: Verfahrenstechnik I, 1972<br>VERLAG CHEMIE, Weinheim/Bergstraße pages 445, 660<br>-- | 1 | **TECHNICAL FIELDS SEARCHED (Int. Cl 4)**<br><br>C 07 D 251/00<br>C 07 B 37/00<br>F 28 D 15/00 | |
| Y | DERWENT JAPANESE PATENTS REPORT, vol. 74, no. 52, January 28, 1975, A: Polymers, page 5<br>DERWENT PUBLICATIONS LTD.<br>& JP-B4-74-047 914 (HAYASHI)<br>-- | 1 | | |
| Y | US - A - 4 092 140 (CERUTTI)<br>* Abstract; column 12, lines 21-37; column 14, lines 39-55 *<br>-- | 1 | | |

| The present search report has been drawn up for all claims | | |
|---|---|---|
| **Place of search**<br>VIENNA | **Date of completion of the search**<br>18-08-1987 | **Examiner**<br>HAMMER |

EPO Form 1503 03 82

## DOCUMENTS CONSIDERED TO BE RELEVANT

EP 87305250.0

| Category | Citation of document with indication, where appropriate, of relevant passages | Relevant to claim | CLASSIFICATION OF THE APPLICATION (Int. Cl.4) |
|---|---|---|---|
| A | <u>DE – A1 – 3 224 766</u> (HENKEL)  * Claim 1 * | 1 | |
| | ---- | | |
| | | | |
| | | | **TECHNICAL FIELDS SEARCHED (Int. Cl.4)** |

The present search report has been drawn up for all claims

| Place of search VIENNA | Date of completion of the search 18-08-1987 | Examiner HAMMER |
|---|---|---|